# EUROPEAN PATENT APPLICATION

(11) **EP 4 582 091 A1**
(43) Date of publication of application: **09.07.2025**
(21) Application number: 24181495.3
(22) Date of filing: 11.06.2024
(51) Int. Cl.: A61K 36/185, A61K 36/484, A61K 36/53, A61K 36/534, A61K 36/539, A61K 36/65, A61K 36/315, A61K 36/575, A61K 36/78, A61K 31/00, A61K 36/238, A61K 36/424, A61K 36/605, A61P 25/08, A61P 25/12, A61P 25/10

(54) **PHARMACEUTICAL COMBINATION FOR PREVENTING OR TREATING EPILEPTIC SEIZURE AND THE USE THEREOF**

(30) Priority: 05.01.2024 TW 113100636
(71) Applicant: Fu Jen Catholic University, New Taipei City, Taiwan 242 (TW)
(72) Inventor: WANG, Su-Jane, 242062 New Taipei City (TW); HUNG, Chi-Feng, 242062 New Taipei City (TW)
(74) Representative: Zimmermann, Tankred Klaus

(57) **Abstract**

The present disclosure provides a traditional Chinese medicine combination for preventing or treating epileptic seizure and use in the preparation a medication. The traditional Chinese medicine combination has the characteristics of high efficiency, safety, and convenience and can effectively prevent or treat epileptic seizure while, and may be further combined with other anti-epileptic drugs to enhance the anti-epileptic effects and reduce medication side effects. The present disclosure also provides a method of preventing or treating epileptic seizure with the traditional Chinese medicine combination or a pharmaceutical combination.

## Description

### BACKGROUND

### Technology Field

The present disclosure relates to a traditional Chinese medicine composition for preventing or treating epileptic seizure and the use thereof. Additionally, the present disclosure also relates to a pharmaceutical combination comprising a traditional Chinese medicine composition and at least one antiepileptic drug and the use thereof.

### Description of Related Art

The background description includes information that may be useful in understanding the present invention. It does not imply that any of the information provided herein constitutes prior art or is relevant to the presently claimed invention, nor does it imply that any publication specifically or implicitly referenced constitutes prior art.

Epilepsy is a common neurological disease that affects as many as 70 million people worldwide. This disorder is characterized by abnormal firing of nerve cells in the brain, primarily in the cerebral cortex and hippocampus, leading to recurrent, spontaneous, and unpredictable seizures. The pathogenesis of epilepsy may be related to congenital or acquired encephalopathy, such as intracerebral trauma, infection, stroke and tumors. However, about 70% of epilepsy patients cannot identify any specific cause of the disease. Although the cause of most epilepsy cases remains unknown, it is generally believed that nerve cells may be overexcited due to an imbalance between the inhibitory nervous systems (i.e., the GABG system) and excitatory nervous systems (i.e., the glutamine system) in the brain.

The mechanism of action of antiepileptic drugs currently used to control epileptic seizure mainly involves directly inhibiting the excitability of nerve cells by blocking sodium ion channels, such as phenytoin, carbamazepine; inhibiting glutamate, such as lamotrigine, felbamate; enhancing GABA, such as benzodiazepines, tiagabine, vigabatrin, to regulate neuronal stability. However, despite the clinically used of nearly thirty drugs to treat epilepsy, approximately one-third of people with epilepsy are resistant to existing medications. In addition, drug side effects also limit patient compliance with existing medications.

Currently known antiepileptic drugs commonly used have many side effects. For example, the possible side effects listed on the package insert of carbazapine include hypersomnia, upset stomach, diarrhea, giddiness, head heaviness, ataxia, aleukemia, aplastic anemia, hepatic insufficiency, arrhythmia, eruption, etc. The possible side effects listed on the package insert of phenytoin are nausea, vomiting, pyrosis, loss of appetite, diplopia, nystagmns, ataxia, gingival hyperplasia, hypertrichosis, rough face, athetosis, lymphadenopathy, monster, eruption, etc. when side effects occur, treatment typically involves changing the medication, reducing the dose, or adding other drugs to suppress the side effects. However, reducing the dose can easily result in epileptic recurrence, causing psychosomatic suffering for patients with epilepsy.

Therefore, finding and developing more effective and safer new drugs is necessary for the treatment of epilepsy. Medicinal plants have attracted attention for their extensive use in traditional and folk medicine for the treatment and prevention of various neurological disorders, including epilepsy. In recent years, many plant-derived bioactive molecules have been confirmed to produce antiepileptic effects by affecting ion channels, GABA or glutamic acid, such as, flavonoids, alkaloids, terpenoids and cannabidiol. Consequently, medicinal plants have become the targets of antiepileptic drug research.

Taiwan Qingguan No. 1 (NRICM101) was developed by the National Institute of Traditional Chinese Medicine in 2020 based on the prescription "Jingfang Baidu Powder" compiled in the Ming Dynasty's "Public Prescription Regimen". It is a traditional Chinese medicine prescription used to treat coronavirus disease (COVID-19) and has antiviral, immunomodulatory and anti-inflammatory effects. NRICM101 is made from ten kinds of Chinese medicinal materials, including scutellaria root (*Scutellaria baicalensis*), heartleaf houttuymia (*Houttuynia cordata*), indigowoad root (*Isatis indigotica*), mongolian snakegourd fruit (*Trichosanthes kirilowii*), mulberry leaf (*Morus alba*), magnolia bark (*Magnolia officinalis*), peppermint herb (*Mentha haplocalyx*), fineleaf nepeta (*Nepeta tenuifolia*), saposhnikovia root (*Saposhnikovia divaricate*), and baked liquorice root (*Glycyrrhiza glabra*). In addition, various bioactive ingredients have also been found in NRICM101, such as baicalin, epigoitrin, liquiritin, quercetin 3-galactoside, quercetin 3-rhamnoside, scutellarin, rutin, oroxindin and caffeoquinic acid. These ingredients may contribute to the pharmacological properties of NRICM101. However, there is currently no available data discussing the anti-epileptic efficacy of NRICM101. This anti-epileptic effect holds the prospect of being a new choice of medication for preventing or treating epileptic seizure, or being used in combination with other anti-epileptic drugs to reduce the side effects of conventional anti-epileptic drugs.

### SUMMARY

The present disclosure provides a traditional Chinese medicine composition for use in the prevention or treatment of epileptic seizure in a patient with epilepsy, wherein the traditional Chinese medicine composition comprises scutellaria root, heartleaf houttuymia, indigowoad root, mongolian snakegourd fruit, mulberry leaf, magnolia bark, peppermint herb, fineleaf nepeta, Saposhnikovia root, and baked liquorice root.

In one embodiment, epilepsy is caused by damage to nerve cells in the brain. In another embodiment, epilepsy is caused by the activation of glial cells. In other embodiment, epilepsy is caused by an inflammatory response in the brain caused by inflammatory molecules. In other embodiment, the inflammatory molecule is selected from the group consisting of 1interleukin-1β (IL-1β), interleukin-6 (IL-6), tumor necrosis factor-α (TNF-α), high mobility group Box 1 (HMGB 1), interleukin-1 receptor 1 (IL-1R1), and Toll-like receptor-4 (TLR-4).

The present disclosure provides a traditional Chinese medicine combination for use in the reduction of the dosage of an antiepileptic drug administrated to a patient with epilepsy, wherein the traditional Chinese medicine combination comprises scutellaria root, heartleaf houttuymia, indigowoad root, mongolian snakegourd fruit, mulberry leaf, magnolia bark, peppermint herb, fineleaf nepeta, saposhnikovia root, and baked liquorice root. In one embodiment, the antiepileptic drug selected from the group of the Western medicine consisting of phenytoin, carbamazepine, lamotrigine, felbamate, benzodiazepines, tiagabine, and vigabatrin.

The present disclosure also provides a pharmaceutical combination for preventing or treating epileptic seizure comprising a traditional Chinese medicine composition and at least one antiepileptic drug and a pharmaceutically acceptable carriers and/or stabilizers, wherein the traditional Chinese medicine composition comprises scutellaria root, heartleaf houttuymia, indigowoad root, mongolian snakegourd fruit, mulberry leaf, magnolia bark, peppermint herb, fineleaf nepeta, Saposhnikovia root, and baked liquorice root, and wherein the dose of the at least one antiepileptic drug when administered in combination with the traditional Chinese medicine combination to prevent or treat epilepsy is lower than the dose when the at least one antiepileptic drug is administered alone. In one embodiment, the at least one antiepileptic drug selected from the group consisting of phenytoin, carbamazepine, lamotrigine, felbamate, benzodiazepines, tiagabine, and vigabatrin. In one embodiment, the traditional Chinese medicine combination and the at least one antiepileptic drug are administered to a subject simultaneously, sequentially or at intervals.

Various objects, features, aspects and advantages of the inventive subject matter will become more apparent from the following detailed description of preferred embodiments.

### BRIEF DESCRIPTION OF THE DRAWINGS

Further features and advantages will become apparent from the following and more particular description of the preferred embodiments of the disclosure, as illustrated in the accompanying drawings, and in which like referenced characters generally refer to the same parts or elements throughout the views, and in which:
Figure 1 shows the phytochemical characteristics chromatogram of NRICM101.
Figure 2 is a histogram of preventing the epileptic behavior of KA-induced animals by pretreatment with NRICM101. Data are means ± SEM (n = 3-8 rats/group). *, p < 0.05; **, p < 0.01; ***, p < 0.001 compared with KA group.
Figures 3 and 4 show the electroencephalogram (EEG) of NRICM101 pretreatment to prevent KA-induced epileptic seizure in animals. Data are means ± SEM (n = 3 rats/group). ***, p < 0.001 compared with control. #, p < 0.05 compared with KA group.

### DETAILED DESCRIPTION OF THE DISCLOSURE

The following description includes information that may be useful in understanding the present invention. It does not imply that any of the information provided herein constitutes prior art or is relevant to the presently claimed invention, nor does it imply that any publication specifically or implicitly referenced constitutes prior art.

At the outset, it is to be understood that this disclosure is not limited to the specifically exemplified materials, architectures, routines, methods or constructions set forth herein. Thus, while numbers options, similar or equivalent to those described herein, can be utilized in the practice or embodiments of this disclosure, the preferred materials and methods are described herein.

The detailed description set forth below in connection with the appended drawings is intended as a description of exemplary embodiments of the present disclosure and is not intended to represent the only exemplary embodiments in which the present disclosure can be practiced. The term "exemplary" used throughout this description means "serving as an example, instance, or illustration," and should not necessarily be construed as preferred or advantageous over other exemplary embodiments. The detailed description includes specific details for the purpose of providing a thorough understanding of the exemplary embodiments of the specification. It will be apparent to those skilled in the art that the exemplary embodiments of the specification may be practiced without these specific details.

### Definitions

As used herein, each of the following terms has the meaning associated with it in this section. It is also understood that the terminology used herein is for the purpose of describing particular embodiments of this disclosure only and is not intended to be limiting.

Generally, unless defined otherwise, all technical and scientific terms used herein have the same meaning as commonly understood by one of ordinary skill in the art to which this invention belongs. The nomenclature used herein and the laboratory procedures in animal pharmacology, pharmaceutical science, separation science, and organic chemistry are those well-known and commonly employed in the art. In the methods described herein, the acts can be carried out in any order, except when a temporal or operational sequence is explicitly recited. Furthermore, specified acts can be carried out concurrently unless explicit claim language recites that they be carried out separately. For example, a claimed act of doing X and a claimed act of doing Y can be conducted simultaneously within a single operation, and the resulting process will fall within the literal scope of the claimed process.

The following non-limiting abbreviations are used herein: KA: kainic acid; IL-1β: interleukin-1β; IL-6: interleukin-6; TNF-α: tumor necrosis factor-α; HMGB1: high mobility group Box 1; IL-1R1: interleukin-1 receptor 1; CBZ: carbamazepine; CABA: gamma-aminobutyric acid; TLR-4: Toll-like receptor-4; and HPLC: high-performance liqid chromatography.

As used in this specification and the appended claims, the singular forms "a", "an" and "the" include plural referents unless the content clearly dictates otherwise. By way of example, "a component" means one component or more than one component.

As used herein, the term "about" will be understood by persons of ordinary skill in the art and will vary to some extent on the context in which it is used. As used herein, when referring to a measurable value such as an amount, a temporal duration, and the like, "about" encompasses variations of ±20%, ±10%, ±5%, ±1%, or ±0.1% from the specified value, as such variations are appropriate to perform the disclosed methods. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. The numerical values presented in some embodiments of the invention may contain certain errors necessarily resulting from the standard deviation found in their respective testing measurements.

In one aspect, the terms "co-administered" and "co-administration" as relating to a subject refer to administering to the subject a traditional Chinese medicine combination of the present disclosure along with other medicaments that may also treat or prevent a disease or disorder contemplated herein. In certain embodiments, the co-administered traditional Chinese medicine combination and other medicaments are administered separately, or in any kind of combination as part of a single therapeutic approach.

As used herein, a "disease" is a state of health of a subject wherein the subject cannot maintain homeostasis, and wherein, if the disease is not ameliorated, then the subject's health continues to deteriorate.

As used herein, a "disorder" in a subject is a state of health in which the subject is able to maintain homeostasis, but in which the subject's state of health is less favorable than it would be in the absence of the disorder. If left untreated, a disorder does not necessarily cause a further decrease in the subject's state of health.

As used herein, the term "pharmaceutically acceptable" refers to a material, such as a carrier or diluent, which does not abrogate the biological activities or properties of the compounds or pharmaceutical ingredients useful within the present disclosure, and is relatively non-toxic, that is, the material may be administered to a subject without causing undesirable biological effects or interacting in a deleterious manner with any of the components of the pharmaceutical combination in which it is contained.

As used herein, a "pharmaceutically effective amount," "therapeutically effective amount," or "effective amount" of a compound or ingredient in the pharmaceutical combination is that amount of compound that is sufficient to provide a beneficial effect to the subject to which it is administered.

As used herein, the terms "subject", "individual" and "patient" can be used interchangeably, and may refer to a human or non-human mammal. Examples of non-human mammals include livestock and pets, such as ovine, porcine, canine, feline and murine mammals. In certain embodiments, the subject is human.

As used herein, the term "traditional Chinese medicine composition" refers to a combination containing at least two or more Chinese medicinal materials with therapeutic effects. Each of the Chinese medicinal materials can be ground and mixed directly, or each of the Chinese medicinal materials may be mixed before or after processing, refining, concentrating and/or other preparation methods according to conventional methods. The term "processing" refers to the process of making medicines from Chinese herbal medicine raw materials, such as fire processing, water processing or water and fire processing methods.

As used herein, the term "pharmaceutical combination" refers to a combination containing at least two or more drugs, which can be Chinese medicine and/or Western medicine.

The term "prevent," "preventing," or "prevention" as used herein means avoiding or delaying the onset of symptoms associated with a disease or condition in a subject who has not developed such symptoms at the time of administering an agent or a pharmaceutical combination commences.

The terms "treat," "treating" and "treatment," as used herein, means reducing the frequency or severity with which symptoms of a disease or condition are experienced by a subject by virtue of administering an agent or a pharmaceutical combination to the subject.

As used herein, the term "therapeutic effectiveness" refers to a favorable treatment response, such as alleviation or improvement of one or more symptoms of a disease; reduction of the severity of the disease; delay or slowdown of disease progression; improvement, reduction or stabilization of the disease state; or other beneficial results.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual and partial numbers within that range, for example, 1, 2, 2.7, 3, 4, 5, 5.3, and 6. This applies regardless of the breadth of the range.

### Traditional Chinese medicine combination

The traditional Chinese medicine composition of the present disclosure is based on NRICM101 and comprises ten kinds of Chinese medicinal materials, namely, scutellaria root, heartleaf houttuymia, indigowoad root, mongolian snakegourd fruit, mulberry leaf, magnolia bark, peppermint herb, fineleaf nepeta, saposhnikovia root, and baked liquorice root. In one embodiment, the traditional Chinese medicine composition consists of scutellaria root, heartleaf houttuymia, indigowoad root, mongolian snakegourd fruit, mulberry leaf, magnolia bark, peppermint herb, fineleaf nepeta, saposhnikovia root, and baked liquorice root.

In one embodiment, the traditional Chinese medicine composition of the present disclosure comprises 3.75-1.25 parts by weight of scutellaria root, 3.75-1.25 parts by weight of heartleaf houttuymia, 3.75-1.25 parts by weight of indigowoad root, 3.75-1.25 parts by weight of mongolian snakegourd fruit, 2.25-0.75 parts by weight of mulberry leaf, 2.25-0.75 parts by weight of magnolia bark, 2.25-0.75 parts by weight of peppermint herb, 2.25-0.75 parts by weight of fineleaf nepeta, 1.5-0.5 parts by weight of saposhnikovia root, and 1.5-0.5 parts by weight of baked liquorice root. In another embodiment, the traditional Chinese medicine composition of the present disclosure preferredly comprises 2.75-2.25 parts by weight of scutellaria root, 2.75-2.25 parts by weight of heartleaf houttuymia, 2.75-2.25 parts by weight of indigowoad root, 2.75-2.25 parts by weight of mongolian snakegourd fruit, 1.65-1.35 parts by weight of mulberry leaf, 1.65-1.35 parts by weight of magnolia bark, 1.65-1.35 parts by weight of peppermint herb, 1.65-1.35 parts by weight of fineleaf nepeta, 1.1-0.9 parts by weight of saposhnikovia root, and 1.1-0.9 parts by weight of baked liquorice root. In other embodiment, the traditional Chinese medicine composition of the present disclosure more preferredly comprises 2.5 parts by weight of scutellaria root, 2.5 parts by weight of Heartleaf Houttuymia, 2.5 parts by weight of indigowoad root, 2.5 parts by weight of mongolian snakegourd fruit, 1.5 parts by weight of mulberry leaf, 1.5 parts by weight of magnolia bark, 1.5 parts by weight of peppermint herb, 1.5 parts by weight of fineleaf nepeta, 1 part by weight of saposhnikovia root, and 1 part by weight of baked liquorice root.

The Chinese medicinal materials contained in the traditional Chinese medicine composition can be made into liquid preparations through steps such as cleaning, drying, grinding, and/or processing. In one embodiment, the liquid preparations can be further made into solid preparations through steps such as extraction, concentration, adding excipients, spray granulation, etc. The above preparation steps can be adjusted according to the dosage form to be prepared according to the conventional pharmaceutical technology in the field of traditional Chinese medicine. In another embodiment, the traditional Chinese medicine combination is processed with water and extracted. In other embodiment, the traditional Chinese medicine combination is processed with water, extracted and dried into power form.

### Combination therapies

In one aspect, the traditional Chinese medicine combination of the present disclosure is useful in combination with one or more agents useful for preventing or treating epileptic seizure. These additional agents may comprise compounds (e.g., commercially available compounds) or pharmaceutical ingredients known to treat, prevent, or reduce the symptoms of epilepsy.

In one embodiment, the present disclosure provides a method of treating or preventing epilepsy in a subject. The method comprises administering to the subjects a therapeutically effective amount of the traditional Chinese medicine combination of the present disclosure. In one embodiment, the subjects are further co-administered with at least one agent that aids in the treatment or prevention of epilepsy. In another embodiment, the at least one agent is selected from, such as, phenytoin, carbamazepine, lamotrigine, felbamate, benzodiazepines, tiagabine, and vigabatrin, wherein the dose of the Western medicine can be adjusted by a doctor according to the recommended dose of each medicine.

In one embodiment, the subject suffering from epilepsy may be a subject who has been diagnosed with epilepsy for the first time or who is taking at least one antiepileptic drug. In one embodiment, the traditional Chinese medicine combination of the present disclosure and at least one antiepileptic drug may be provided to the subject diagnosed with epilepsy for the first time, wherein the dose of the at least one antiepileptic drug when administered in combination with the traditional Chinese medicine combination of the present disclosure is lower than the dose when the at least one antiepileptic drug is administered alone for preventing or treating epilepsy. In another embodiment, the traditional Chinese medicine combination of the present disclosure can further be provided to the subjects who are already taking at least one antiepileptic drug, and the dose of the at least one antiepileptic drug can be further reduced.

### Administration/Dosing

The regimen of administration may affect what constitutes an effective amount. The pharmaceutical combination may be administered to the patient either prior to or after the onset of a disease or disorder. Additionally, several divided doses, as well as staggered doses, may be administered daily or sequentially. Furthermore, the doses of ingredients in the pharmaceutical combination may be proportionally increased or decreased as indicated by the exigencies of the therapeutic or prophylactic situation.

Administration of the traditional Chinese medicine combination of the present disclosure to a patient may be carried out using known procedures, at dosages and for periods of time effective to treat or prevent epilepsy. An effective amount of the traditional Chinese medicine combination necessary to achieve the effect on treating or preventing epilepsy may vary according to factors, such as the activity of the traditional Chinese medicine combination employed; the time of administration; the rate of excretion of the extract; the duration of the treatment; and other medicines, compounds, or materials used in combination with the extract. It may also depend on the state of the disease or disorder, as well as age, sex, weight, condition, general health and prior medical history of the patient being treated, and similar factors well-known in the medical arts. Dosage regimens may be adjusted to provide the optimum therapeutic response. For example, several divided doses may be administered daily or the dose may be proportionally reduced as indicated by the exigencies of the therapeutic situation.

Although the descriptions of the traditional Chinese medicine combination provided herein are principally directed to the traditional Chinese medicine combination suitable for ethical administration to humans, it will be understood by the skilled artisan that such traditional Chinese medicine combination are generally suitable for administration to various animals as well. Modifying the combination suitable for administration to humans in order to render the traditional Chinese medicine combination suitable for administration to various animals is well understood, and the ordinarily skilled veterinary pharmacologist can design and perform such modification with merely ordinary, if any, experimentation. Subjects to which the administration of the traditional Chinese medicine combination of the present disclosure is contemplated include, but are not limited to, humans, other primates, and mammals including commercially relevant mammals, such as bovine, porcine, equine, ovine, feline, and canine.

In certain embodiments, the traditional Chinese medicine combination of the present disclosure is administered to the patient in dosages that range from one to five times per day or more. In other embodiments, the traditional Chinese medicine combination of the present disclosure are administered to the patient in range of dosages that include, but are not limited to, once every day, every two days, every three days to once a week, and once every two weeks. It will be readily apparent to one skilled in the art that the frequency of administration of the various combinations of the present disclosure will vary from subject to subject depending on many factors including, but not limited to, age, the severity of disease or disorder to be treated, gender, overall health, and other factors. Thus, the invention should not be construed to be limited to any particular dosage regime and the precise dosage and combination to be administered to any patient will be determined by the attending physician taking all other factors about the patient into account.

In one embodiment, when administering the traditional Chinese medicine combination of the present disclosure and at least one antiepileptic drug, the traditional Chinese medicine combination and the at least one antiepileptic drug can be administered to the subject at the same time. In another embodiment, the traditional Chinese medicine combination and the at least one antiepileptic drug are administered to the subject simultaneously, sequentially or at intervals.

### Administration

### Oral Administration

The dosage form of the traditional Chinese medicine combination of the present disclosure for oral administration may be in solid or liquid form. Suitable solid dosage forms include, for example, powders, pellets, granules, pills, tablets, capsules, troches, and the like. Suitable liquid dosage forms include, for example, solutions, syrups, suspensions, dispersions, concentrates, and the like. These dosage forms can be prepared by conventional pharmaceutical methods, optionally adding pharmaceutically acceptable additives.

Those skilled in the art will recognize, or be able to ascertain using no more than routine experimentation, numerous equivalents to the specific procedures, embodiments, claims, and examples described herein. Such equivalents were considered to be within the scope of the present disclosure and covered by the claims appended hereto. For example, it should be understood, that modifications in reaction conditions, including but not limited to reaction times, reaction size/volume, and experimental reagents, such as solvents, catalysts, pressures, atmospheric conditions, and reducing/oxidizing agents, with art-recognized alternatives and using no more than routine experimentation, are within the scope of the present disclosure.

It is to be understood that, wherever values and ranges are provided herein, the description in range format is merely for convenience and brevity and should not be construed as an inflexible limitation on the scope of the present disclosure. Accordingly, all values and ranges encompassed by these values and ranges are meant to be encompassed within the scope of the present disclosure. Moreover, all values that fall within these ranges, as well as the upper or lower limits of a range of values, are also contemplated by the present disclosure. The description of a range should be considered to have specifically disclosed all the possible sub-ranges as well as individual numerical values within that range and, when appropriate, partial integers of the numerical values within ranges. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

The following examples further illustrate aspects of the present disclosure. However, they are in no way a limitation of the teachings or disclosure of the present disclosure as set forth herein.

### EXAMPLES

The present disclosure is now described with reference to the following Examples. These Examples are provided for the purpose of illustration only, and the present disclosure is not limited to these Examples, but rather encompasses all variations that are evident as a result of the teachings provided herein.

### Materials & Methods

The following procedures can be utilized in preparing and/or testing exemplary traditional Chinese medicine combination of the present disclosure.

### Traditional Chinese medicine combination

In the traditional Chinese medicine combinations (NRICM101, Sun Ten Pharmaceutical Co., Ltd., Taiwan) used in the following examples, one powder formula contains 2.5 parts by weight of scutellaria root, 2.5 parts by weight of heartleaf houttuymia, 2.5 parts by weight of indigowoad root, 2.5 parts by weight of mongolian snakegourd fruit, 1.5 parts by weight of mulberry leaf, 1.5 parts by weight of magnolia bark, 1.5 parts by weight of peppermint herb, 1.5 parts by weight of fineleaf nepeta, 1 part by weight of saposhnikovia root, and 1 part by weight of baked liquorice root.

### Experimental animals

Male Sprague-Dawley rats (SD rat, 150-200 g) were purchased from BioLASCO (Taiwan) and bred in Fu Jen Laboratory Animal Center. All animal experiments were approved by the IACUC of Fu Jen Catholic University. According to the 3R principles, all experiments were performed to minimize animal suffering and use the minimum number of animals necessary to produce reliable results.

### Example 1 - Ingredient analysis of NRICM101

Regarding the phytochemical characteristics of NRICM101, some standard compounds were selected from literature disclosures, and some active compounds were identified in the methanol extracts. The structures of these compounds were confirmed through 3D fingerprint analysis.

For high performance liquid chromatography (HPLC) analysis, 0.5 g NRICM101 was extracted using 20 mL of 70% methanol with ultrasonic shaking at 25°C for 20 minutes. The NRICM101 sample was then filtered through a 0.45 µm syringe filter.

The Waters HPLC system (Milford, MA, USA) consisted of a Waters 600 pump system, a Waters 2996 photodiode array detector, a Waters 717 plus autosampler, and a Sugai U-620 column oven (Japan, Wakayama City). Cosmosil 5C18-MS-II reversed-phase chromatography column (5 µm, 4.6 mm × 250 mm, Nacalai tesque, Japan) is equipped with Lichrospher RP-18 end-capped guard column (5 µm, 4.0 mm × 10 mm, Merck, Germany) as the stationary phase. The gradient eluent consists of Eluent A (H₂O/KH₂PO₄/10% H₃PO₄ = 1000 mL/2.72 g/1 mL), Eluent B (acetonitrile) and Eluent C (H₂O). Gradient elution is performed according to the following curve:
- 0-30 minutes, 90%-75% of Eluent A and 10%-25% of Eluent B;
- 30-40 minutes, 75%-65% of Eluent A and 25%-35% of Eluent B;
- 40-55 minutes, 65%-0% of Eluent A, 35%-75% of Eluent B and 0%-25% of Eluent C;
- 55-60 minutes, 75%-10% of Eluent B and 25%-90% of Eluent C; and
- 60-65 minutes, 0%-90% of Eluent A, 10%-10% of Eluent B and 90%-0% of Eluent C.
Gradient elution is used for 3D fingerprint analysis of liquiritin (25.94 min, 280 nm), rosmarinic acid (29.44 min, 320 nm), baicalin (34.61 min, 280 nm), oroxylin A-7-O-glucuronide (40.39 min, 280 nm), wogonin-7-O-glucuronide (42.53 min, 280 nm), glycyrrhizic acid (51.37 min, 250 nm), magnolol (61.79 min, 290 nm). The flow rate was 1 mL/min, and the column temperature was maintained at 35°C.

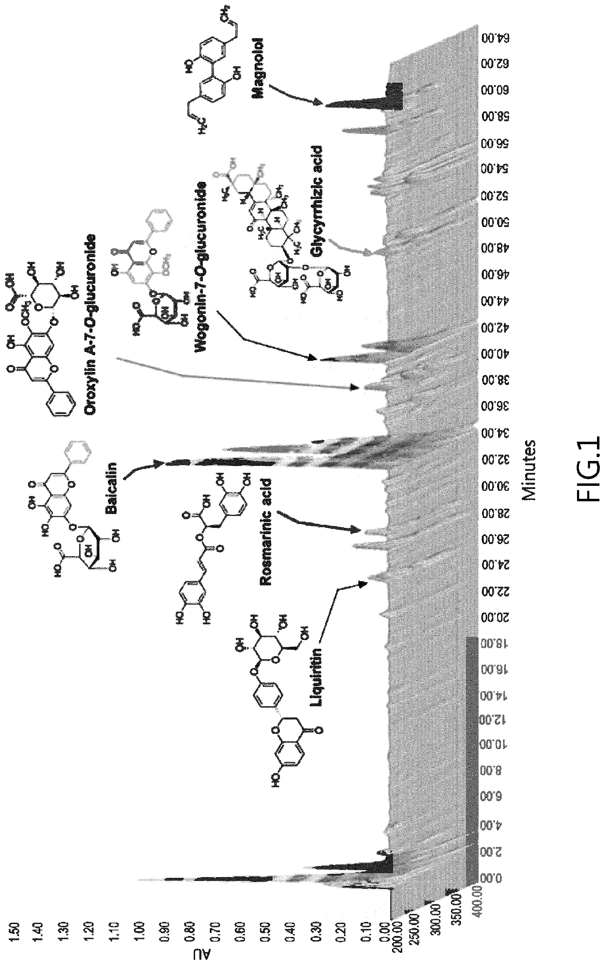

Figure 1 shows the phytochemical characteristics chromatogram of NRICM101. In the freeze-dried extract of NRICM101, the contents of baicalin, oroxylin A-7-O-glucuronide, wogonin-7-O-glucuronide, glycyrrhizic acid, liquiritin, magnolol, and rosmarinic acid are 18.34, 1.33, 2.67, 2.42, 1.70, 1.07 and 0.91 mg/g, respectively.

### Example 2 - KA-induced epilepsy animal model

The antiepileptic effect of the traditional Chinese medicine combination was evaluated using kainic acid (KA)-induced epilepsy model in animals. KA is an analogue of glutamate, and injection of KA into rats will increase the release of glutamate and overactivate glutamate receptors, resulting in progressive limbic seizures in rats. This phenomenon leads to increased calcium ions in nerve cells, oxidative stress, and loss of mitochondrial function, resulting in the death of nerve cells in many areas of the brain, especially in the cerebral cortex and hippocampus. In addition, KA-induced neuronal cell death is related to glial cell activation and inflammatory response caused by inflammatory molecules, such as interleukin-1β (IL-1β), interleukin-6 (IL-6), tumor necrosis factor-α (TNF-α), high mobility group Box 1 (HMGB1), interleukin 1 receptor 1 (IL-1R1), and Toll-like receptor-4 (TLR-4). Since these pathological changes induced by KA are similar to the behavior and pathology of human epilepsy, the KA-induced epilepsy model in animals has been widely used to screen new compounds with antiepileptic activity.

Epileptic behavioral expressions were analyzed within 4 hours after intraperitoneal injection of KA in rats. Seizures were assessed according to the Racine scale (1972), as shown in the table below.

| Seizure stage | Behavioral expression |
|---|---|
| 1 | facial clonus |
| 2 | nodding and wet dog shaking |
| 3 | forelimb clonus |
| 4 | forelimb clonus with rearing |
| 5 | rearing, jumping, and falling |

### Example 3 - Evaluation of the effect of the traditional Chinese medicine combination on KA-induced epileptic behavior in animals

In order to evaluate the effect of the traditional Chinese medicine combination on KA-induced epileptic behavior in animals, rats in each group were administrated physiological saline, the traditional Chinese medicine composition or CBZ through feeding tubes regularly every day, and then intraperitoneally injected KA (15 mg/kg) seven days later, wherein the traditional Chinese medicine composition and CBZ are formulated as aqueous solutions. The epileptic behavioral performance was analyzed within 4 hours after intraperitoneal injection of KA in each rat, and the epileptic behavioral performance was evaluated according to the above-mentioned Racine scale (1972).

Sprague-Dawley rats were divided into the following groups according to the administration of different test substances:
1. Negative control group: normal saline
2. KA group: No test article was administered before injection of kainic acid (KA)
3. Low-dose group of the traditional Chinese medicine combination: 100 mg/kg of the traditional Chinese medicine combination
4. Medium-dose group of the traditional Chinese medicine combination: 200 mg/kg of the traditional Chinese medicine combination
5. High-dose group of the traditional Chinese medicine combination: 300 mg/kg of the traditional Chinese medicine combination
6. Antiepileptic drug group: 100 mg/kg of CBZ
7. Antiepileptic pharmaceutical combination group: 100 mg/kg of the traditional Chinese medicine combination and 50 mg/kg of CBZ

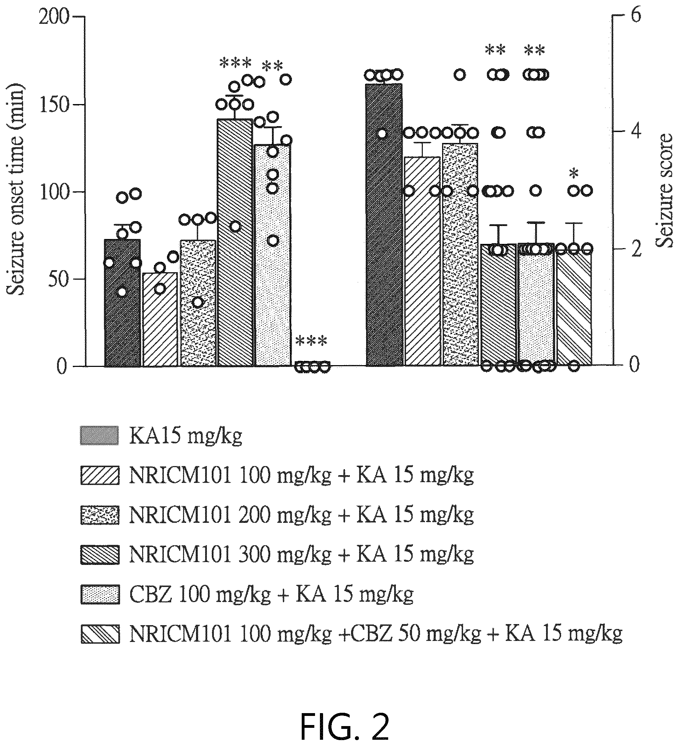

Rats in each group were administrated test substances through feeding tubes at regular intervals every day, wherein the control group was administrated normal saline, the traditional Chinese medicine combination groups were administrated the traditional Chinese medicine combination, the antiepileptic drug group was administrated CBZ, and the antiepileptic pharmaceutical combination group was administrated the traditional Chinese medicine combination and CBZ. The traditional Chinese medicine combination and CBZ were ground into powder, and then mixed with normal saline before administering to rats. Seven days later, except for the negative control group, rats in each group were injected intraperitoneally with KA (15 mg/kg/i.p. each time). The dose of KA injection was based on previous studies. The epileptic behavior of rets was analyzed within 3 hours after intraperitoneal injection of KA. The results are shown in Figure 2.

As shown in Figure 2, compared with the KA group, the high-dose group (300 mg/kg) of the traditional Chinese medicine combination of the present disclosure showed a significant effect (p < 0.001). In addition, the number of seizure-free animals was highest (77%, p<0.001) in the high-dose group of the traditional Chinese medicine combination, followed by the groups treated with the medium-dose group of the traditional Chinese medicine combination (33%, p > 0.05) or the low-dose group of the traditional Chinese medicine combination (40%, p > 0.05). At the same time, the impact of the high-dose group of the traditional Chinese medicine combination on epileptic seizure time and epilepsy scores was similar to that of the antiepileptic drug group (100 mg/kg CBZ) (p > 0.05). This result proves that the traditional Chinese medicine combination of the present disclosure has the effect of preventing epileptic seizure caused by KA. In other words, the traditional Chinese medicine combination of the present disclosure can be used instead of antiepileptic drugs.

In addition, in the Antiepileptic pharmaceutical combination group (100 mg/kg of the traditional Chinese medicine combination and 50 mg/kg of CBZ), the epileptic seizure time and severity of the animals were significantly improved compared with the KA (15 mg/kg) group (p < 0.05). In essence, the antiepileptic pharmaceutical combination of the present disclosure can greatly improve the shortcoming of low-dose of antiepileptic drugs not effectively suppressing the symptoms of epilepsy. Since the antiepileptic pharmaceutical combination group can produce the effect of preventing KA-induced seizures similar to that of the antiepileptic drug group, this result shows that the combination of low-dose of the traditional Chinese medicine combination (100 mg/kg) and low-dose CBZ (50 mg/kg) can be used to prevent KA-induced seizures in animals, and such low doses of CBZ can further reduce the side effects of antiepileptic drug.

In this experiment with rats, it is confirmed that the method of the present disclosure can be used to prevent seizures, especially for refractory epileptic symptoms. When used alone, the traditional Chinese medicine combination of the present disclosure can provide antiepileptic effects comparable to those of antiepileptic drugs (such as CBZ). In other words, the traditional Chinese medicine combination of the present disclosure can be used instead of antiepileptic drugs. In addition, when the traditional Chinese medicine combination is used in combination with antiepileptic drugs (such as CBZ), the antiepileptic effects can still be achieved with further reduction of the dose of antiepileptic drugs. Thus, whether the traditional Chinese medicine combination of the present disclosure is used alone or in combination with antiepileptic drugs, the side effects of antiepileptic drugs can be reduced. The method provided by the present disclosure has lower toxicity and side effects, making it a safe and effective method for preventing epilepsy.

### Example 4 - Evaluation of the effect of traditional Chinese medicine combination on KA-induced epileptic behavior in animals through electroencephalogram (EEG) recordings

The rats were divided into groups as in Example 3 above. The control group was administrated normal saline, the traditional Chinese medicine combination groups were administrated the traditional Chinese medicine combination, the antiepileptic drug group was administrated CBZ, and the antiepileptic pharmaceutical combination group was administrated the traditional Chinese medicine combination and CBZ. Seven days later, except for the negative control group, rats in each group were injected intraperitoneally with KA (15 mg/kg/i.p. each time). The dose of KA injection was based on previous studies. Electroencephalogram (EEG) recordings of epileptic seizures were analyzed within 3 hours after intraperitoneal injection of KA.

EEG recordings were obtained using a three-channel EEG system (Pinnacle Technology Inc., Lawrence, Kansas, USA) according to previously described methods (ref.). After being anesthetized with 3% isoflurane, the rats were fixed in a stereotactic frame (RWD, Life. Science, Dover, USA) and four electrodes were surgically implanted. Two electrodes were placed on either side of the frontal cortex relative to bregma (anteroposterior (AP), + 3.9; medial (M), ± 2.0), and two electrodes were placed on the parietal cortex (AP -6.4, ML ± 4.0). The surgery was performed under isoflurane anesthesia. One week post-surgery, the rats were connected to an EEG recording amplifier (Mode #8213-SE3), and EEG signals were recorded using a data acquisition system (Pinnacle Technology Inc., Lawrence, Kansas, USA) over a period of 3 hours. Electrical signals from the brain are pre-amplification through an eight-channel probe (HS-8-CNR-MDR50, Neuralynx). The signal was then digitized at 1.6 kHz and band-pass filtered online between 0.1 and 100Hz using Cheetah 6.2.0 recording software. The criteria for identifying recorded events as epileptic seizure are high-amplitude, rhythmic discharges, including repetitive spikes, spike-wave discharges, and slow waves, with a duration of at least 10 seconds. The number and duration of EEG epileptic seizure were analyzed using PAL-8200EEG software (Pinnacl Technologies).

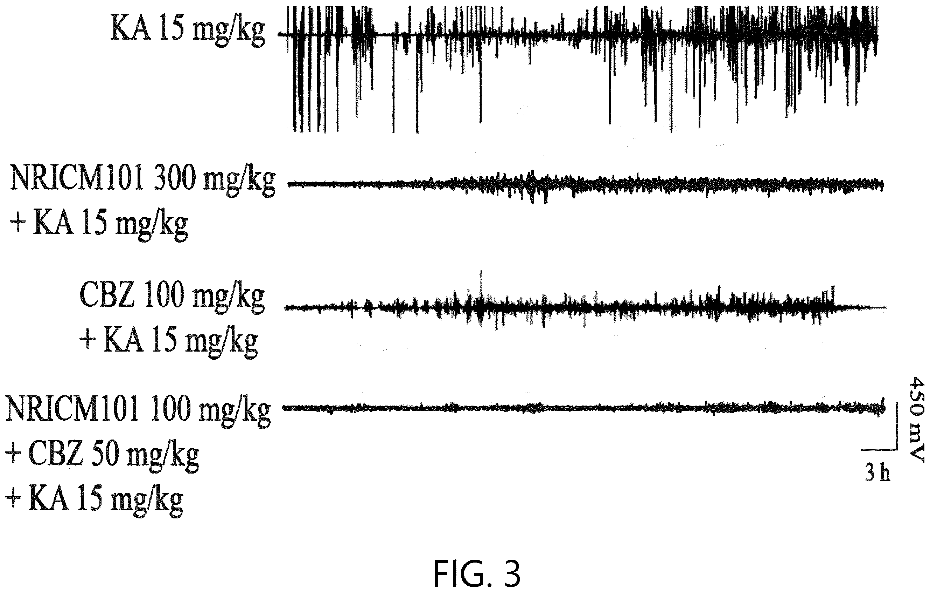

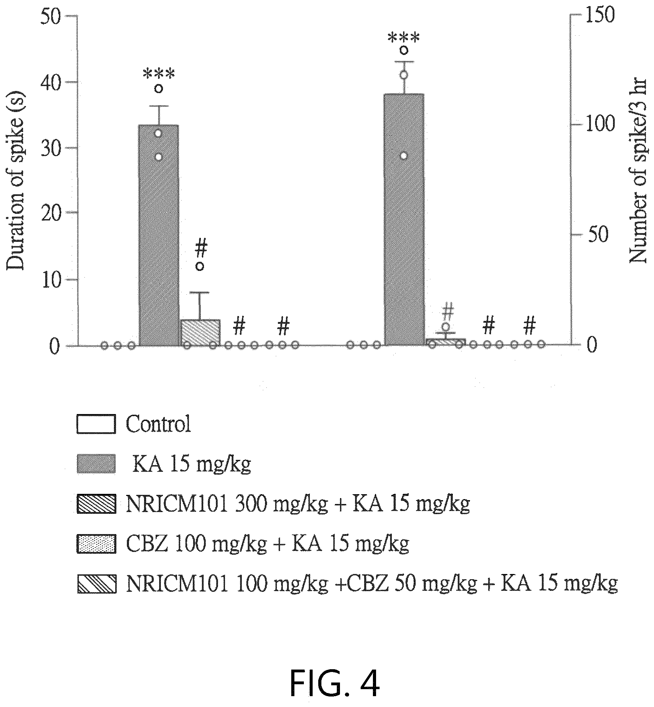

EEG recordings were used to evaluate epileptic seizure peaks in the rat brain. As shown in Figures 3 and 4, KA treatment significantly increased the number and duration of epileptic seizure peaks compared with controls. However, compared with the KA group, the number and duration of epileptic seizures in the high-dose group of the traditional Chinese medicine combination (300 mg/kg of the traditional Chinese medicine combination) and the antiepileptic pharmaceutical combination group (100 mg/kg of the traditional Chinese medicine combination and 50 mg/kg of CBZ) were reduced. This effect was also similar to the antiepileptic drug group (100 mg/kg of CBZ) (p > 0.05). These results prove that the traditional Chinese medicine combination of the present disclosure and the pharmaceutical combination of the traditional Chinese medicine combination and antiepileptic drug have the effect of preventing epilepsy caused by KA. In addition, when the traditional Chinese medicine combination of the present disclosure is used in combination with an antiepileptic drug, the dose of the antiepileptic drug (such as CBZ) can be further reduced while still achieving the anti-epileptic effect.

The results of the above animal experiments prove that the traditional Chinese medicine combination of the present disclosure can significantly prolong the inhibition time of KA-induced seizure and reduce the severity of KA-induced seizure, which proves that the traditional Chinese medicine combination has the effect of preventing epilepsy. Therefore, the traditional Chinese medicine combination of the present disclosure has the prospect of being used in the development of antiepileptic drugs and/or related products.

### Enumerated Embodiments:

The following exemplary embodiments are provided, the numbering of which is not to be construed as designating levels of importance.

Embodiment 1 provides a traditional Chinese medicine combination for use in the prevention or treatment of epileptic seizure, wherein the traditional Chinese medicine combination comprises scutellaria root (*Scutellaria baicalensis*), heartleaf houttuymia (*Houttuynia cordata*), indigowoad root (*Isatis indigotica*), mongolian snakegourd fruit (*Trichosanthes kirilowii*), mulberry leaf (*Morus alba*), magnolia bark (*Magnolia officinalis*), peppermint herb (*Mentha haplocalyx*), fineleaf nepeta (*Nepeta tenuifolia*), saposhnikovia root (*Saposhnikovia divaricate*), and baked liquorice root (*Glycyrrhiza glabra*)*.*

Embodiment 2 provides the traditional Chinese medicine combination for use according to Embodiment 1, wherein the traditional Chinese medicine combination comprises 3.75-1.25 parts by weight of scutellaria root, 3.75-1.25 parts by weight of heartleaf houttuymia, 3.75-1.25 parts by weight of indigowoad root, 3.75-1.25 parts by weight of mongolian snakegourd fruit, 2.25-0.75 parts by weight of mulberry leaf, 2.25-0.75 parts by weight of magnolia bark, 2.25-0.75 parts by weight of peppermint herb, 2.25-0.75 parts by weight of fineleaf nepeta, 1.5-0.5 parts by weight of saposhnikovia root, and 1.5-0.5 parts by weight of baked liquorice root.

Embodiment 3 provides the traditional Chinese medicine combination for use according to Embodiment 1, wherein the traditional Chinese medicine combination comprises 2.75-2.25 parts by weight of scutellaria root, 2.75-2.25 parts by weight of heartleaf houttuymia, 2.75-2.25 parts by weight of indigowoad root, 2.75-2.25 parts by weight of mongolian snakegourd fruit, 1.65-1.35 parts by weight of mulberry leaf, 1.65-1.35 parts by weight of magnolia bark, 1.65-1.35 parts by weight of peppermint herb, 1.65-1.35 parts by weight of fineleaf nepeta, 1.1-0.9 parts by weight of saposhnikovia root, and 1.1-0.9 parts by weight of baked liquorice root.

Embodiment 4 provides the the traditional Chinese medicine combination for use according to Embodiment 1, wherein the traditional Chinese medicine combination comprises 2.5 g of scutellaria root, 2.5 g of heartleaf houttuymia, 2.5 g of indigowoad root, 2.5 g of mongolian snakegourd fruit, 1.5 g of mulberry leaf, 1.5 g of magnolia bark, 1.5 g of peppermint herb, 1.5 g of fineleaf nepeta, 1 g of saposhnikovia root, and 1 g of baked liquorice root.

Embodiment 5 provides the traditional Chinese medicine combination for use according to Embodiment 1, wherein the traditional Chinese medicine combination is processed with water and extracted.

Embodiment 6 provides the traditional Chinese medicine combination for use according to Embodiment 5, wherein the traditional Chinese medicine combination is further dried into power form.

Embodiment 7 provides the traditional Chinese medicine combination for use according to Embodiment 1, wherein the epileptic seizure is caused by damage to nerve cells in the brain.

Embodiment 8 provides the traditional Chinese medicine combination for use according to Embodiment 1, wherein the epileptic seizure is caused by the activation of glial cells.

Embodiment 9 provides the traditional Chinese medicine combination for use according to Embodiment 1, wherein the epileptic seizure is caused by an inflammatory response in the brain caused by inflammatory molecules.

Embodiment 10 provides the traditional Chinese medicine combination for use according to Embodiment 9, wherein the inflammatory molecule is selected from the group consisting of interleukin-1β (IL-1β), interleukin-6 (IL-6), tumor necrosis factor-α (TNF-α), high mobility group Box 1 (HMGB1), interleukin-1 receptor 1 (IL-1R1), and Toll-like receptor-4 (TLR-4).

Embodiment 11 provides the traditional Chinese medicine combination for use according to Embodiment 1, wherein the traditional Chinese medicine combination is further combined with at least one antiepileptic drug.

Embodiment 12 provides the traditional Chinese medicine combination for use according to Embodiment 11, wherein the at least one antiepileptic drug is selected from the group of consisting of phenytoin, carbamazepine, lamotrigine, felbamate, benzodiazepines, tiagabine, and vigabatrin.

Embodiment 13provides the traditional Chinese medicine combination for use according to Embodiment 12, wherein the at least one antiepileptic drug is carbamazepine.

Embodiment 14 provides the traditional Chinese medicine combination for use according to Embodiment 1, wherein the traditional Chinese medicine combination is used in the treatment or prevention of epileptic seizure in human.

Embodiment 15 provides the traditional Chinese medicine combination for use according to Embodiment 1, wherein the traditional Chinese medicine combination is used in the prevention or treatment of epileptic seizure in non-human mammals.

Embodiment 16 provides a pharmaceutical combination for preventing or treating epileptic seizure comprising a traditional Chinese medicine combination and at least one antiepileptic drug, wherein the traditional Chinese medicine combination comprises scutellaria root, heartleaf houttuymia, indigowoad root, mongolian snakegourd fruit, mulberry leaf, magnolia bark, peppermint herb, fineleaf nepeta, saposhnikovia root, and baked liquorice root, and the traditional Chinese medicine combination is processed with water and extracted.

Embodiment 17 provides the pharmaceutical combination of Embodiment 16, wherein the traditional Chinese medicine combination comprises 3.75-1.25 parts by weight of scutellaria root, 3.75-1.25 parts by weight of heartleaf houttuymia, 3.75-1.25 parts by weight of indigowoad root, 3.75-1.25 parts by weight of mongolian snakegourd fruit, 2.25-0.75 parts by weight of mulberry leaf, 2.25-0.75 parts by weight of magnolia bark, 2.25-0.75 parts by weight of peppermint herb, 2.25-0.75 parts by weight of fineleaf nepeta, 1.5-0.5 parts by weight of saposhnikovia root, and 1.5-0.5 parts by weight of baked liquorice root.

Embodiment 18 provides the pharmaceutical combination of Embodiment 16, wherein the traditional Chinese medicine combination comprises 2.75-2.25 parts by weight of scutellaria root, 2.75-2.25 parts by weight of heartleaf houttuymia, 2.75-2.25 parts by weight of indigowoad root, 2.75-2.25 parts by weight of mongolian snakegourd fruit, 1.65-1.35 parts by weight of mulberry leaf, 1.65-1.35 parts by weight of magnolia bark, 1.65-1.35 parts by weight of peppermint herb, 1.65-1.35 parts by weight of fineleaf nepeta, 1.1-0.9 parts by weight of saposhnikovia root, and 1.1-0.9 parts by weight of baked liquorice root.

Embodiment 19 provides the pharmaceutical combination of Embodiment 16, wherein the traditional Chinese medicine combination comprising 2.5 g of scutellaria root, 2.5 g of heartleaf houttuymia, 2.5 g of indigowoad root, 2.5 g of mongolian snakegourd fruit, 1.5 g of mulberry leaf, 1.5 g of magnolia bark, 1.5 g of peppermint herb, 1.5 g of fineleaf nepeta, 1 g of saposhnikovia root, and 1 g of baked liquorice root.

Embodiment 20 provides the pharmaceutical combination of Embodiment 16, wherein the dose of the at least one antiepileptic drug in the pharmaceutical combination is lower than the dose used by the at least one antiepileptic drug alone to prevent or treat epileptic seizure.

Embodiment 21 provides the pharmaceutical combination of Embodiment 16, wherein the at least one antiepileptic drug is selected from the group of consisting of phenytoin, carbamazepine, lamotrigine, felbamate, benzodiazepines, tiagabine, and vigabatrin.

Embodiment 22 provides the pharmaceutical combination of Embodiment 16, wherein the at least one antiepileptic drug is carbamazepine.

Embodiment 23 provides the pharmaceutical combination of Embodiment 16, wherein the traditional Chinese medicine combination and the at least one antiepileptic drug are administered to a subject simultaneously, sequentially or at intervals.

Embodiment 24 provides a traditional Chinese medicine combination for use in the reduction of the dosage of an antiepileptic drug administrated to a patient with epilepsy, wherein the traditional Chinese medicine combination comprises scutellaria root, heartleaf houttuymia, indigowoad root, mongolian snakegourd fruit, mulberry leaf, magnolia bark, peppermint herb, fineleaf nepeta, saposhnikovia root, and baked liquorice root, and the traditional Chinese medicine combination is processed with water and extracted.

Embodiment 25 provides the traditional Chinese medicine combination for use according to Embodiment 24, wherein the traditional Chinese medicine combination is further dried into power form.

Embodiment 26 provides the traditional Chinese medicine combination for use according to Embodiment 24, wherein the traditional Chinese medicine combination comprises 3.75-1.25 parts by weight of scutellaria root, 3.75-1.25 parts by weight of heartleaf houttuymia, 3.75-1.25 parts by weight of indigowoad root, 3.75-1.25 parts by weight of mongolian snakegourd fruit, 2.25-0.75 parts by weight of mulberry leaf, 2.25-0.75 parts by weight of magnolia bark, 2.25-0.75 parts by weight of peppermint herb, 2.25-0.75 parts by weight of fineleaf nepeta, 1.5-0.5 parts by weight of saposhnikovia root, and 1.5-0.5 parts by weight of baked liquorice root.

Embodiment 27 provides the traditional Chinese medicine combination for use according to Embodiment 24, wherein the antiepileptic drug is at least one selected from the group of consisting of phenytoin, carbamazepine, lamotrigine, felbamate, benzodiazepines, tiagabine, and vigabatrin.

Embodiment 28 provides the traditional Chinese medicine combination for use according to Embodiment 27, wherein the antiepileptic drug is carbamazepine.

Embodiment 29 provides the traditional Chinese medicine combination for use according to Embodiment 24, wherein the traditional Chinese medicine combination and the antiepileptic drug are administered to the patient simultaneously, sequentially or at intervals.

Embodiment 30 provides the traditional Chinese medicine combination for use according to Embodiment 24, wherein the patient is a mammal.

Embodiment 31 provides the traditional Chinese medicine combination for use according to Embodiment 24, wherein the patient is a human.

While this invention has been disclosed with reference to specific embodiments, it is apparent that other embodiments and variations of this invention may be devised by others skilled in the art without departing from the true spirit and scope of the invention. The appended claims are intended to be construed to include all such embodiments and equivalent variations.

## Claims

1. A pharmaceutical combination for use in the prevention or treatment of epileptic seizure comprising a traditional Chinese medicine combination, wherein the traditional Chinese medicine combination comprises 3.75-1.25 parts by weight of scutellaria root (*Scutellaria baicalensis*), 3.75-1.25 parts by weight of heartleaf houttuymia (*Houttuynia cordata*), 3.75-1.25 parts by weight of indigowoad root (*Isatis indigotica*), 3.75-1.25 parts by weight of mongolian snakegourd fruit (*Trichosanthes kirilowii*), 2.25-0.75 parts by weight of mulberry leaf (*Morus alba*), 2.25-0.75 parts by weight of magnolia bark (*Magnolia officinalis*), 2.25-0.75 parts by weight of peppermint herb (*Mentha haplocalyx*), 2.25-0.75 parts by weight of fineleaf nepeta (*Nepeta tenuifolia*), 1.5-0.5 parts by weight of saposhnikovia root (*Saposhnikovia divaricate*), and 1.5-0.5 parts by weight of baked liquorice root (*Glycyrrhiza glabra*), and the traditional Chinese medicine combination is processed with water and extracted.

2. The pharmaceutical combination for use according to claim 1, wherein the traditional Chinese medicine combination is further dried into power form.

3. The pharmaceutical combination for use according to claim 1, wherein the epileptic seizure is caused by damage to nerve cells in the brain.

4. The pharmaceutical combination for use according to claim 1, wherein the epileptic seizure is caused by the activation of glial cells.

5. The pharmaceutical combination for use according to claim 1, wherein the epileptic seizure is caused by an inflammatory response in the brain caused by inflammatory molecules.

6. The pharmaceutical combination for use according to claim 5, wherein the inflammatory molecule is selected from the group consisting of interleukin-1β (IL-1β), interleukin-6 (IL-6), tumor necrosis factor-α (TNF-α), high mobility group Box 1 (HMGB1), interleukin-1 receptor 1 (IL-1R1), and Toll-like receptor-4 (TLR-4).

7. The pharmaceutical combination for use according to claim 1, wherein the pharmaceutical combination further comprises at least one antiepileptic drug, and the dose of the at least one antiepileptic drug is lower than the dose used by the at least one antiepileptic drug alone to prevent or treat epileptic seizures.

8. The traditional Chinese medicine combination for use according to claim 7, wherein the at least one antiepileptic drug is selected from the group of consisting of phenytoin, carbamazepine, lamotrigine, felbamate, benzodiazepines, tiagabine, and vigabatrin.

9. The traditional Chinese medicine combination for use according to claim 7, wherein the traditional Chinese medicine combination and the at least one antiepileptic drug are administered to a patient simultaneously, sequentially or at intervals.

10. The pharmaceutical combination for use according to claim 9, wherein the patient is a mammal.

11. A traditional Chinese medicine combination for use in the reduction of the dosage of an antiepileptic drug administrated to a patient with epilepsy, wherein the traditional Chinese medicine combination comprises 3.75-1.25 parts by weight of scutellaria root, 3.75-1.25 parts by weight of heartleaf houttuymia, 3.75-1.25 parts by weight of indigowoad root, 3.75-1.25 parts by weight of mongolian snakegourd fruit, 2.25-0.75 parts by weight of mulberry leaf, 2.25-0.75 parts by weight of magnolia bark, 2.25-0.75 parts by weight of peppermint herb, 2.25-0.75 parts by weight of fineleaf nepeta, 1.5-0.5 parts by weight of saposhnikovia root, and 1.5-0.5 parts by weight of baked liquorice root, and the traditional Chinese medicine combination is processed with water and extracted.

12. The traditional Chinese medicine combination for use according to claim 11, wherein the traditional Chinese medicine combination is further dried into power form.

13. The traditional Chinese medicine combination for use according to claim 11, wherein the antiepileptic drug is at least one selected from the group of consisting of phenytoin, carbamazepine, lamotrigine, felbamate, benzodiazepines, tiagabine, and vigabatrin.

14. The traditional Chinese medicine combination for use according to claim 11, wherein the traditional Chinese medicine combination and the antiepileptic drug are administered to the patient simultaneously, sequentially or at intervals.

15. The traditional Chinese medicine combination for use according to claim 11, wherein the patient is a mammal.
